# FASCICULE DE BREVET EUROPEEN

(11) **EP 1 097 145 B1**
(45) Date de publication et mention de la délivrance du brevet: **28.05.2003**
(21) Numéro de dépôt: 99929482.0
(22) Date de dépôt: 13.07.1999
(51) Int. Cl.: C07D 241/12, A61K 31/495

(54) **DERIVES DE POLYHYDROXYALKYLPYRAZINE ET LEUR PREPARATION ET MEDICAMENTS LES CONTENANT**
POLYHYDROXYALKYLPYRAZINE DERIVATE UND DEREN HERSTELLUNG UND DIESE ENTHALTENDE ARZNEIMITTEL
POLYHYDROALKYLPYRAZINE DERIVATIVES AND THEIR PREPARATION AND MEDICINES CONTAINING THEM

(30) Priorité: 16.07.1998 FR 9809088
(43) Date de publication de la demande: 09.05.2001
(73) Titulaire: Aventis Pharma S.A., 92160 Antony (FR)
(72) Inventeur: BASHIARDES, Georges, F-94320 Thiais (FR); CARRY, Jean-Christophe, F-92190 Meudon (FR); EVERS, Michel, F-04510 La Queue en Brie (FR); FILOCHE, Bruno, F-92290 Créteil (FR); MIGNANI, Serge, F-92290 Chatenay Malabry (FR)
(86) Numéro de dépôt international: FR9901719
(87) Numéro de publication internationale: WO00004002

(56) Documents cités:
- WO-A-97/28813
- CHEMICAL ABSTRACTS, vol. 90, no. 11, 1979 Columbus, Ohio, US; abstract no. 19276z, page 329; XP002060269 cité dans la demande & JP 07 890401 A (JAPAN TOBACCO)

## Description

La présente invention concerne les médicaments contenant en tant que principe actif au moins un composé de formule: 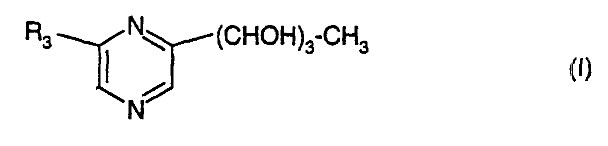 ou un de ses stéréoisomères ou un de ses sels avec un acide minéral ou organique, les nouveaux composés de formule (I), leurs stéréoisomères, leurs sels avec un acide minéral ou organique et leur préparation.

Dans la formule (I)
R₃ représente une chaîne -CH₂-(CHOH)₂-CH₃.

Les composés de formule (I) comportant plusieurs carbones asymétriques, présentent des formes stéréoisomères. Ces différents stéréoisomères font partie de l'invention.

Les médicaments selon l'invention préférés sont ceux qui contiennent comme ingrédient actif au moins un composé de formule (I) choisi parmi :
1-[6-(2R,3S-dihydroxy-butyl)-pyrazin-2-yl]-butane-1S,2S,3S-triol
1-[6-(2R,3R-dihydroxy-butyl)-pyrazin-2-yl]-butane-1S,2S,3R-triol
1-[6-(2S,3S-dihydroxy-butyl)-pyrazin-2-yl]-butane-1S,2R,3S-triol
1-[6-(2S,3R-dihydroxy-butyl)-pyrazin-2-yl]-butane-1S,2R,3R-triol
1-[6-(2R,3S-dihydroxy-butyl)-pyrazin-2-yl]-butane-1R,2S,3S-triol
1-[6-(2R,3R-dihydroxy-butyl)-pyrazin-2-yl]-butane-1R,2S,3R-triol
1-[6-(2S,3S-dihydroxy-butyl)-pyrazin-2-yl]-butane-1R,2R,3S-triol
1-[6-(2S,3R-dihydroxy-butyl)-pyrazin-2-yl]-butane-1R,2R,3R-triol
et leurs sels avec un acide minéral ou organique.

Les médicaments particulièrement préférés sont ceux qui contiennent en tant que principe actif au moins un composé de formule (I) choisi parmi les suivants :
1-[6-(2S,3S-dihydroxy-butyl)-pyrazin-2-yl]-butane-1S,2R,3S-triol
1-[6-(2R,3R-dihydroxy-butyl)-pyrazin-2-yl]-butane-1R,2S,3R-triol
1-[6-(2R,3S-dihydroxy-butyl)-pyrazin-2-yl]-butane-1S,2S,3S-triol
et leurs sels avec un acide minéral ou organique.

Les composés de formule (I) suivante sont connus (brevet JP78-90401):

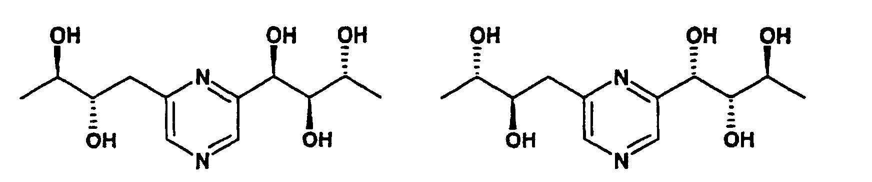

Aucune activité pharmacologique n'est décrite pour tous ces dérivés.

Les autres composés de formule (I), leurs stéréoisomères et leurs sels avec un acide minéral ou organique sont nouveaux et font également partie de l'invention.

Les composés de formule (I) préférés sont les composés suivants :
1-[6-(2S,3S-dihydroxy-butyl)-pyrazin-2-yl]-butane-1S,2R,3S-triol
1-[6-(2R,3R-dihydroxy-butyl)-pyrazin-2-yl]-butane-1R,2S,3R-triol
et leurs sels avec un acide minéral ou organique.

Les composés de formule (I) peuvent être préparés par action de formiate d'ammonium sur un ou deux aldoses de formule :

H₃C-(CHOH)₃-CHOH-CHO (II)

ou un de ses stéréoisomères.

Cette réaction s'effectue généralement en milieu aqueux, à une température comprise entre 20°C et 100°C.

Les aldoses H₃C-(CHOH)₃-CHOH-CHO et leurs stéréoisomères sont commercialisés ou peuvent être préparés à partir :
a) d'aldoses commercialement disponibles :
   - par des réactions d'épimérisation par application ou adaptation des méthodes décrites dans Adv. Carbohydr. Chem., 13, 63, (1958) notamment en milieu basique au moyen d'une solution aqueuse diluée de soude (0,03 à 0.05%), à une température comprise entre 20 et 40°C,
   - par des réactions d'allongement de chaîne par application ou adaptation des méthodes décrites dans « The Carbohydrates», éditeurs: W. Pigman et D. Horton, Academic Press, New-York, Volume IA, 133 (1972) et notamment en formant la cyanhydrine de l'aldose de départ (par exemple par action du cyanure de sodium en solution aqueuse, à une température comprise entre 10 et 30°C et en présence de soude, à un pH voisin de 9) puis hydrolyse de la fonction nitrile ainsi formée en acide correspondant par application ou adaptation des méthodes décrites dans Organic Synthesis volume I page 436 et volume III page 85 (par exemple à l'aide d'acide chlorhydrique ou d'acide sulfurique concentré, en solution aqueuse, à une température comprise entre 20°C et la température d'ébullition du milieu réactionnel), puis réduction de la fonction acide carboxylique en aldéhyde correspondant par application ou adaptation des méthodes décrites dans J. Am. Chem. Soc. 71, 122 (1949) notamment à l'aide d'un borohydrure d'un métal alcalin (le borohydrure de sodium par exemple), en solution aqueuse à une température comprise entre 20°C et la température d'ébullition du milieu réactionnel,
      par des réactions de racourcissement de chaînes par application ou adaptation des méthodes décrites dans «The Carbohydrates», éditeurs: W. Pigman et D. Horton, Academic Press, New-York, Volume IB, 1980, page 929 ou Chem. Ber., 83, 559 (1950) et notamment en transformant la fonction aldéhyde de l'aldose en hydroxylamine correspondant par application ou adaptation des méthodes décrites dans Organic Synthesis volume II page 314 (par exemple à l'aide de chlorhydrate d'hydroxylamine, en solution aqueuse et en présence d'une base telle que le carbonate de sodium à une température comprise entre 20 et 50°C), puis action du 3,4-dinitrofluorobenzène en présence de dioxyde de carbone et d'une base telle le d'hydrogénocarbonate de sodium en solution aqueuse et d'un alcool aliphatique (alcool isopropylique par exemple), à une température comprise entre 50 et 80°C,
   - par des réactions de désoxygénation par application ou adaptation des méthodes décrites dans Carbohydr. Res., 36, 392, (1974) et 28D, 357, (1996) notamment en transformant une fonction alcool de l'aldose en sulfonate correspondant par application ou adaptation des méthodes décrites dans Carbohydr. Res., 54, 105, (1977) et dans J. Carbohydr. Chem., 6, 169, (1987) et 6, 537, (1987), par exemple à l'aide de chlorure de méthanesulfonyle ou de p-toluènesulfonyle et en présence d'une base, puis action de borhydrure de sodium ou d'aluminohydrure de lithium dans un solvant respectivement tel que le diméthylsulfoxyde d'une part, ou le benzène et l'éther diéthylique d'autre part, à une température comprise entre 20°C et la température d'ébullition du milieu réactionnel,
b) d'alcools allyliques correspondants par application ou adaptation des méthodes décrites dans Science, 220, 949 (1983) et notamment à l'aide d'hydroperoxyde de terbutyle en présence d'un complexe de titane (IV) tel que le complexe isopropylate de titane (IV) et tartrate de dialkyle optiquement pur (le tartrate de diéthyle par exemple), suivi de l'action successive de thiophénolate de sodium, d'acide para-chloroperbenzoïque dans l'anhydride acétique et d'hydrure de diisopropylaluminium.

Les mélanges réactionnels obtenus par les divers procédés décrits précédemment sont traités suivant des méthodes classiques physiques (évaporation, extraction, distillation, chromatographie, cristallisation par exemple) ou chimiques (formation de sels par exemple).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré.

Ces sels font également partie de l'invention.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate.

Les exemples suivants illustrent l'invention :

### EXEMPLE 1

Une solution de 10,0 g de L-fucose et 19,2 g de formiate d'ammonium dans 30 cm³ d'eau est chauffée à reflux pendant 6 heures puis laissée refroidir à température ambiante. Le mélange est concentré sous pression réduite (2,7 kPa) à une température de 50°C. Le résidu pâteux marron est repris dans 50 cm³ d'éthanol, trituré, filtré et la fraction insoluble est lavée à l'éthanol (opération répétée une fois). Le filtrat est concentré sous pression réduite (2,7 kPa) à une température de 50°C pour donner une pâte brune qui est purifiée par chromatographie sur une colonne de silice (0,020-0,045 mm) à une pression d'environ 1,5x10⁵ Pa en éluant avec un mélange chloroforme/méthanol/solution aqueuse d'ammoniac (12/6/1 en volumes). Les fractions contenant le produit attendu sont réunies et concentrées sous pression réduite (2,7 kPa) à une température de 50°C. La pâte brune obtenue est reprise dans 3 cm³ d'éthanol puis est reconcentrée sous pression réduite (2,7 kPa) à une température voisine de 50°C pour donner une pâte marron. Après lyophilisation, on obtient 1,23 g de 1-[6-(2S,3S-dihydroxy-butyl)-pyrazin-2-yl]-butane-1S,2R,3S-triol sous forme d'un solide beige [Spectre de R.M.N. ¹H (400 MHz, (CD₃)₂SO d6, δ en ppm) : 1,10 et 1,12 (2 d, J = 6 Hz, 3H chacun : CH₃ 2d et CH₃ 6d); 2,76 et 2,91 (2 dd, respectivement J = 14 et 9 Hz et J = 14 et 3 Hz, 1H chacun : CH₂ 6α); 3,49 (mt, 1H : CH 2β); 3,59 (mt, 1H : CH 6γ); 3,70 (mt, 1H : CH 6β); 3,86 (mt, 1H : CH 2y); 4,31 (d large, J = 5 Hz, 1H : OH en 2y); 4,42 (d, J = 7 Hz, 1H : OH en 2β); 4,55 (mt, 2H : OH en 6b et OH en 6y); 4,61 (dd, J = 7 et 5Hz, 1H : CH 2α); 5,49 (d large, J = 5 Hz, 1H : OH en 2α); 8,40 (s, 1H : =CH en 5); 8,48 (s, 1H : =CH en 3). a_{D}²⁰= -37,6° +/-1,0 (c=0,5/ méthanol)].

### EXEMPLE 2

Une solution de 2 g de α-L-rhamnose et 7 g de formiate d'ammonium dans 8 cm³ d'eau est chauffée à reflux pendant 0,5 heure puis laissée refroidir à température ambiante. Le mélange est filtré puis le filtrat est concentrée sous pression réduite (2,7 kPa) à une température de 45°C. Par trois fois, le résidu est repris par de l'éther diéthylique et évaporé. Le solide pâteux ainsi obtenu est extrait deux fois avec 200 cm³ d'acétone. Les solutions sont concentrées sous pression réduite (2,7 kPa) à une température de 45°C, les résidus sont repris dans 100 cm³ d'éthanol, filtrés et les solutions sont réunies. Le filtrat est concentré sous pression réduite (2,7 kPa) à une température de 45°C, puis le résidu est repris par du dichlorométhane. Le précipité est filtré pour donner un solide brun. Celui-ci est chromatographié sur une colonne de silice (0,040-0,063 mm) éluée avec un mélange acétate d'éthyle/methanol/acide acétique (6/1/1 en volumes).

A - Les fractions contenant du produit de rf. voisin de 0,3 sont réunies et concentrées sous pression réduite (2,7 kPa) à une température de 40°C. L'huile ainsi obtenue est chromatographiée de nouveau sur une colonne de silice (0,040-0,063 mm) éluée avec un mélange acétate d'éthyle/éthanol/solution aqueuse d'ammoniac/eau (40/10/2/2 en volumes). Les fractions contenant le produit attendu sont réunies et concentrées sous pression réduite (2,7 kPa) à une température de 40°C. L'huile ainsi obtenue est triturée dans de l'éther diéthylique puis reprise dans un peu d'éthanol et le précipité qui se forme est filtré. On isole ainsi 64 mg de 1-[6-(2R,3S-dihydroxy-butyl)-pyrazin-2-yl]-butane-1S,2S,3S-triol sous forme d'une poudre blanche [Spectre de R.M.N. ¹H (400 MHz, (CD₃)₂SO d6, δ en ppm) : 1,13 (d, J = 6,5 Hz, 3H : CH₃ 6δ); 1,16 (d, J = 6,5 Hz, 3H : CH₃ 2δ); 2,66 et 3,04 (2 dd, respectivement J = 14 et 9 Hz et J = 14 et 4 Hz, 1H chacun : CH₂ 6α); de 3,30 à 3,40 (mt: 1H correspondant au CH 2β); 3,48 (mt, 1H : CH 6y); 3,58 (mt, 1H : CH 6β); 3,71 (mt, 1H: CH 2y); 4,44 (d, J = 8 Hz, 1H : OH en 2β); de 4,60 à 4,70 (mit, 3H : OH en 6β - OH en 6γ et OH en 2y); 4,45 (d large, J = 6,5 Hz, 1H : CH 2α); 5,28 (d, J = 6,5 Hz, 1H : OH en 2α); 8,34 (s, 1H : =CH en 5); 8,55 (s, 1H : =CH en 3).

### EXEMPLE 3

Une solution de 3,28 g de D-fucose et 6,31 g de formiate d'ammonium dans 12 cm³ d'eau est chauffée à reflux pendant 2 heures puis laissée refroidir à température ambiante. Le mélange est concentré sous pression réduite (2,7 kPa) à une température de 55°C. Le résidu pâteux est repris dans 70 cm³ d'éthanol, trituré, filtré et la fraction insoluble est lavée à l'éthanol (opération répétée trois fois). Le filtrat est concentré sous pression réduite (2,7 kPa) à une température voisine de 50°C pour donner une huile brune (1,8 g) qui est purifiée par chromatographie sur une colonne de silice (0,020-0,045 mm) en éluant avec un mélange chloroforme/méthanol/solution aqueuse d'ammoniac (12/6/1 en volumes) en récoltant des fractions de 50 cm³. Les fractions contenant le produit attendu sont réunies et concentrées sous pression réduite (2,7 kPa) à une température de 50°C. La pâte brune obtenue est chromatographiée une seconde fois dans les mêmes conditions Les fractions contenant le produit attendu sont réunies et concentrées sous pression réduite (2,7 kPa) à une température de 50°C. L'huile obtenue est reprise dans 8 cm³ d'eau distillée, la solution est filtrée. Après lyophilisation, on obtient 0,25 g de 1-[6-(2R,3R-dihydroxy-butyl)-pyrazin-2-yl]-butane-1R,2S,3R-triol sous forme d'un solide beige [Spectre de R.M.N. ¹H (400 MHz, (CD₃)₂SO d6, à une température de 353 K, δ en ppm) : 1,14 (d, J = 7 Hz, 3H : CH₃ 6δ); 1,16 (d, J = 7 Hz, 3H : CH₃ 2δ); 2,82 et 2,97 (2 dd, respectivement J = 14 et 9 Hz et J = 14 et 4 Hz, 1H chacun : CH₂ 6α); 3,56 (mt, 1H : CH2β); 3,63 (mt, 1H : CH6γ); 3,76 (mt, 1H : CH6β); 3,88 (mt, 1H: CH2γ); de 3,90 à 4,30 (mf, 4H : OH); 4,67 (d, J = 7 Hz, 1H: CH2α); 5,18 (mf, 1H : OH); 8,41 (s, 1H : =CH en 5); 8,52 (s, 1H : =CH en 3)].

Les composés de formule (I) présentent des propriétés pharmacologiques intéressantes. Ce sont des hypoglycémiants.

L'activité hypoglycémiante des composés de formule (I) a été déterminée sur la réponse hyperglycémique à l'administration de glucose par la voie orale chez la souris normoglycémique, selon le protocole suivant :
Des souris Swiss albinos pesant entre 22 et 26 g sont laissées à jeun pendant 2 heures. A la fin de cette période, la glycémie est mesurée et, immédiatement après, une dose de glucose (2 g/kg) est administrée par voie orale. Trente minutes plus tard, la glycémie est mesurée encore une fois. Les souris qui répondent par une hyperglycémie supérieure à 170 mg/dl sont sélectionnées et utilisées pour détecter l'activité hypoglycémiante des composés selon l'invention.
Les souris ainsi choisies sont réparties en groupes d'au moins 10 animaux. Des groupes distincts reçoivent une solution de 3 à 50 mg/kg du produit à tester dans un véhicule tel que l'eau ou un mélange de méthylcellulose/tween et eau une fois par jour par tubage gastrique. Le traitement dure 4 jours. Au 4 ^{ème} jour, après le dernier traitement, les animaux reçoivent une dose de glucose (2 g/kg) et la glycémie est mesurée 20 à 40 minutes plus tard. Le pourcentage d'inhibition de la réponse hyperglycémique à l'administration de glucose est calculée par rapport à la réponse mesurée dans le groupe traité par le véhicule.

Dans ce test, les composés selon l'invention présentent un pourcentage d'inhibition de la glycémie supérieur ou égal à 10%.

Les composés de formule générale (I) selon l'invention présentent une faible toxicité. Leur DL50 est supérieure à 2000 mg/kg par voie orale chez la souris.

En thérapeutique humaine, ces produits sont utiles dans la prévention et le traitement du diabète et notamment du diabète de type II (NID diabète), du diabète de l'obèse, du diabète de la cinquantaine, du diabète métapléthorique, du diabète du sujet âgé et du diabète léger. lls peuvent être utilisés en complément de l'insulinothérapie dans le diabète insulino dépendant où ils permettent de diminuer progressivement la dose d'insuline, le diabète instable, le diabète insulinorésistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation et thrombose capillaire, microanévrismes, shunt artérioveineux, dilatation veineuse, hémorragies ponctiformes et maculaires, exudats, oedèmes maculaires, manifestations de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies, neuropathies autonomes, amyotrophies diabétiques), les manifestations du pied diabétique (ulcéres des extrémités inférieures et du pied), la néphropathie diabétique dans ses deux formes diffuse et nodulaire, l'athéromatose (élévation des HDL lipoproteines favorisant l'élimination du cholestérol à partir des plaques d'athérome, baisse des LDL lipoproteines, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéines A et B), de la cataracte, de l'hypertension artérielle et ses conséquences.

Les médicaments selon l'invention sont constitués par un composé selon l'invention ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

L'invention concerne également l'utilisation des composés de formule générale (I), leurs stéréoisomères et leurs sels avec un acide minéral ou organique pour la préparation de compositions pharmaceutiques utiles pour le traitement ou la prévention du diabète et les complications du diabète.

## Revendications

1. Médicaments contenant en tant que principe actif au moins un composé de formule: 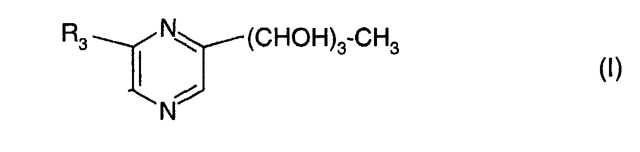 dans laquelle
R₃ représente une chaîne -CH₂-(CHOH)₂-CH₃
ou un de ses stéréoisomères ou un de ses sels avec un acide minéral ou organique pharmaceutiquement acceptable.

2. Médicaments selon la revendication 1 contenant en tant que principe actif au moins un composé choisi parmi les suivants :
1-[6-(2R,3S-dihydroxy-butyl)-pyrazin-2-yl]-butane-1S,2S,3S-triol
1-[6-(2R,3R-dihydroxy-butyl)-pyrazin-2-yl]-butane-1S,2S,3R-triol
1-[6-(2S,3S-dihydroxy-butyl)-pyrazin-2-yl]-butane-1S,2R,3S-triol
1-[6-(2S,3R-dihydroxy-butyl)-pyrazin-2-yl]-butane-1S,2R,3R-triol
1-[6-(2R,3S-dihydroxy-butyl)-pyrazin-2-yl]-butane-1R,2S,3S-triol
1-[6-(2R,3R-dihydroxy-butyl)-pyrazin-2-yl]-butane-1R,2S,3R-triol
1-[6-(2S,3S-dihydroxy-butyl)-pyrazin-2-yl]-butane-1R,2R,3S-triol
1-[6-(2S,3R-dihydroxy-butyl)-pyrazin-2-yl]-butane-1R,2R,3R-triol
ou un de leurs stéréoisomères et un de leurs sels avec un acide minéral ou organique pharmaceutiquement acceptable.

3. Médicaments selon la revendication 1 contenant en tant que principe actif au moins un composé choisi parmi les suivants :
1-[6-(2S,3S-dihydroxy-butyl)-pyrazin-2-yl]-butane-1S,2R,3S-triol
1-[6-(2R,3R-dihydroxy-butyl)-pyrazin-2-yl]-butane-1R,2S,3R-triol
1-[6-(2R,3S-dihydroxy-butyl)-pyrazin-2-yl]-butane-1S,2S,3S-triol
ou un de leurs sels avec un acide minéral ou organique pharmaceutiquement acceptable.

4. Composés de formule : 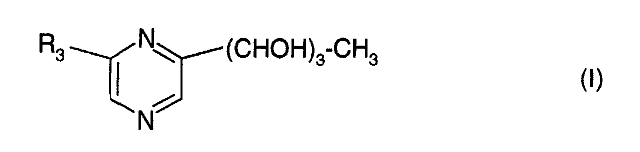 dans laquelle
R₃ représente une chaîne -CH₂-(CHOH)₂-CH₃ à l'exception des produits suivants: 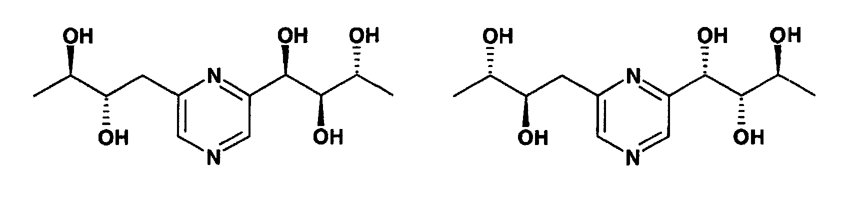 ou un de ses stéréoisomères ou un de ses sels avec un acide minéral ou organique

5. Composés de formule (I) selon la revendication 4 choisis parmi :
1-[6-(2R,3R-dihydroxy-butyl)-pyrazin-2-yl]-butane-1S,2S,3R-triol
1-[6-(2S,3S-dihydroxy-butyl)-pyrazin-2-yl]-butane-1S,2R,3S-triol
1-[6-(2S,3R-dihydroxy-butyl)-pyrazin-2-yl]-butane-1S,2R,3R-triol
1-[6-(2R,3 S-dihydroxy-butyl)-pyrazin-2-yl]-butane-1R,2S,3S-triol
1-[6-(2R,3R-dihydroxy-butyl)-pyrazin-2-yl]-butane-1R,2S,3R-triol
1-[6-(2S,3 S-dihydroxy-butyl)-pyrazin-2-yl]-butane-1R,2R,3S-triol
ou un sel de ces composés avec un acide minéral ou organique.

6. Procédé de préparation des composés de formule (I) selon la revendication 4 **caractérisé en ce que** l'on fait réagir du formiate d'ammonium sur un ou deux aldoses de formule :
H₃C-(CHOH)₃-CHOH-CHO (II)
isole le produit et le transforme éventuellement en sel avec un acide minéral ou organique.

7. Utilisation des composés de formule : 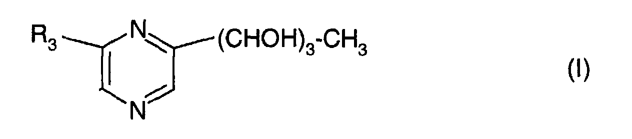 dans laquelle
R₃ représente une chaîne -CH₂-(CHOH)₂-CH₃ stéréoisomères ou un de leurs sels avec un acide minéral ou organique pour la préparation de compositions pharmaceutiques utiles pour le traitement ou la prévention du diabète et les complications du diabète.

## Patentansprüche

1. Arzneimittel, enthaltend als Wirkstoff mindestens eine Verbindung der allgemeinen Formel (I) 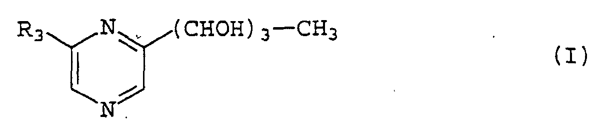 in der
R₃ eine Kette -CH₂-(CHOH)₂-CH₃ darstellt, oder eines ihrer Stereoisomeren oder eines ihrer Salze mit einer pharmazeutisch akzeptablen Mineralsäure oder organischen Säure.

2. Arzneimittel nach Anspruch 1, enthaltend als Wirkstoff mindestens eine der unter den folgenden ausgewählten Verbindungen:
1-[6-(2R,3S-Dihydroxy-butyl)-pyrazin-2-yl]-butan-1S,2S,3S-triol,
1-[6-(2R,3R-Dihydroxy-butyl)-pyrazin-2-yl]-butan-1S,2S,3R-triol,
1-[6-(2S,3S-Dihydroxy-butyl)-pyrazin-2-yl]-butan-1S,2R,3S-triol,
1-[6-(2S,3R-Dihydroxy-butyl)-pyrazin-2-yl]-butan-1S,2R,3R-triol,
1-[6-(2R,3S-Dihydroxy-butyl)-pyrazin-2-yl]-butan-1R,2S,3S-triol,
1-[6-(2R,3R-Dihydroxy-butyl)-pyrazin-2-yl]-butan-1R,2S,3R-triol,
1-[6-(2S,3S-Dihydroxy-butyl)-pyrazin-2-yl]-butan-1R,2R,3S-triol,
1- [6-(2S,3R-Dihydroxy-butyl)-pyrazin-2-yl]-butan-1R,2R,3R-triol
oder eines ihrer Stereoisomeren und eines ihrer Salze mit einer pharmazeutisch akzeptablen Mineralsäure oder organischen Säure.

3. Arzneimittel nach Anspruch 1, enthaltend als Wirkstoff mindestens eine der unter den folgenden ausgewählten Verbindungen:
1-[6-(2S,3S-Dihydroxy-butyl)-pyrazin-2-yl]-butan-1S,2R,35-triol,
1-[6-(2R,3R-Dihydroxy-butyl)-pyrazin-2-yl]-butan-1R,2S,3R-triol,
1-[6-(2R,3S-Dihydroxy-butyl)-pyrazin-2-yl]-butan-1S,2S,3S-triol
oder eines ihrer Salze mit einer pharmazeutisch akzeptablen Mineralsäure oder organischen Säure.

4. Verbindungen der Formel (I) 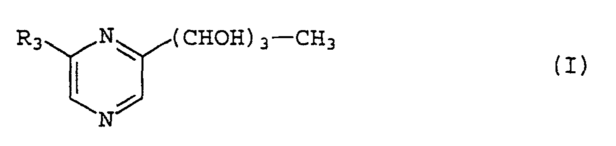 in der R₃ eine Kette -CH₂-(CHOH)₂-CH₃ darstellt, mit Ausnahme der folgenden Produkte: 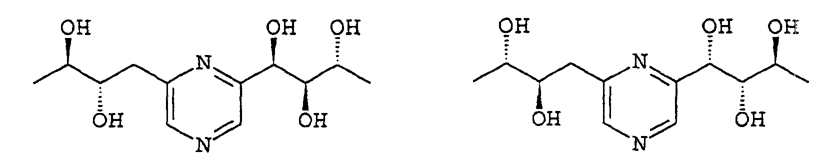 oder eines ihrer Stereoisomeren oder eines ihrer Salze mit einer Mineralsäure oder organischen Säure.

5. Verbindungen der Formel (I) nach Anspruch 4, ausgewählt unter
1-[6-(2R,3R-Dihydroxy-butyl)-pyrazin-2-yl]-butan-1S,2S,3R-triol,
1-[6-(2S,3S-Dihydroxy-butyl)-pyrazin-2-yl]-butan-1S,2R,3S-triol,
1-[6-(2S,3R-Dihydroxy-butyl)-pyrazin-2-yl]-butan-1S,2R,3R-triol,
1-[6-(2R,3S-Dihydroxy-butyl)-pyrazin-2-yl]-butan-1R,2S,3S-triol,
1-[6-(2R,3R-Dihydroxy-butyl)-pyrazin-2-yl]-butan-1R,2S,3R-triol,
1-[6-(2S,3S-Dihydroxy-butyl)-pyrazin-2-yl]-butan-1R,2R,3S-triol
oder ein Salz dieser Verbindungen mit einer Mineralsäure oder organischen Säure.

6. Verfahren zur Herstellung der Verbindungen der Formel (I) nach Anspruch 4, **dadurch gekennzeichnet, daß** man Ammoniumformiat mit einer oder mit zwei Aldosen der Formel (II)
H₃C-(CHOH)₃-CHOH-CHO (II)
zur Reaktion bringt, das Produkt isoliert und gegebenenfalls in ein Salz mit einer Mineralsäure oder organischen Säure überführt.

7. Verwendung der Verbindungen der Formel (I) 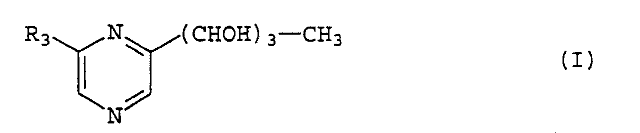 in der
R₃ eine Kette -CH₂-(CHOH)₂-CH₃ darstellt, ihrer Stereoisomeren oder von einem ihrer Salze mit einer Mineralsäure oder organischen Säure für die Herstellung von pharmazeutischen Zusammensetzungen zur Behandlung oder Vorbeugung von Diabetes und von Komplikationen von Diabetes.

## Claims

1. Medicaments comprising, as active principle, at least one compound of formula: 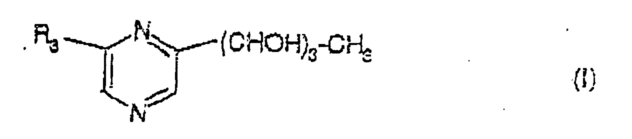 in which
R₃ represents a -CH₂-(CHOH)₂-CH₃ chain,
or one of its stereoisomers or one of its salts with a pharmaceutically acceptable inorganic or organic acid.

2. Medicaments according to Claim 1 comprising, as active principle, at least one compound chosen from the following:
1-[6-(2R,3S-dihydroxybutyl)pyrazin-2-yl]butane-1S,2S,3S-triol
1-[6-(2R,3R-dihydroxybutyl)pyrazin-2-yl]butane-1S,2S,3R-triol
1-[6-(2S,3S-dihydroxybutyl)pyrazin-2-yl]butane-1S,2R,3S-triol
1-[6-(2S,3R-dihydroxybutyl)pyrazin-2-yl]butane-1S,2R,3R-triol
1-[6-(2R,3S-dihydroxybutyl)pyrazin-2-yl]butane-1R,2S,3S-triol
1-[6-(2R,3R-dihydroxybutyl)pyrazin-2-yl]butane-1R,2S,3R-triol
1-[6-(2S,3S-dihydroxybutyl)pyrazin-2-yl]butane-1R,2R,3S-triol
1-[6-(2S,3R-dihydroxybutyl)pyrazin-2-yl]butane-1R,2R,3R-triol
or one of their stereoisomers and one of their salts with a pharmaceutically acceptable inorganic or organic acid.

3. Medicaments according to Claim 1 comprising, as active principle, at least one compound chosen from the following:
1-[6-(2S,3S-dihydroxybutyl)pyrazin-2-yl]butane-1S,2R,3S-triol
1-[6-(2R,3R-dihydroxybutyl)pyrazin-2-yl]butane-1R,2S,3R-triol
1-[6-(2R,3S-dihydroxybutyl]pyrazin-2-yl]butane-1S,2S,3S-triol
or one of their salts with a pharmaceutically acceptable inorganic or organic acid.

4. Compounds of formula: 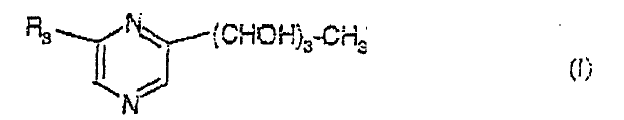 in which
R₃ represents a -CH₂-(CHOH)₂-CH₃ chain, with the exception of the following products: 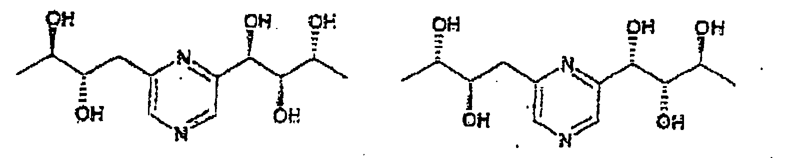 or one of its stereoisomers or one of its salts with an inorganic or organic acid.

5. Compounds of formula (I) according to Claim 4 chosen from:
1-[6-(2R,3R-dihydroxybutyl)pyrazin-2-yl]butane-1S,2S,3R-triol
1-[6-(2S,3S-dihydroxybutyl)pyrazin-2-yl]butane-1S,2R,3S-triol
1- [6- (2S,3R-dihydroxybutyl)pyrazin-2-yl]butane-1S,2R,3R-triol
1-[6-(2R,3S-dihydroxybutyl)pyrazin-2-yl]butane-1R,2S,3S-triol
1- [6- (2R,3R-dihydroxybutyl)pyrazin-2-yl]butane-1R,2S,3R-triol
1-[6-(2S,3S-dihydroxybutyl)pyrazin-2-yl]butane-1R,2R,3S-triol
or a salt of these compounds with an inorganic or organic acid.

6. Process for the preparation of the compounds of formula (I) according to Claim 4, **characterized in that** ammonium formate is reacted with one or two aldoses of formula:
H₃C-(CHOH)₃-CHOH-CHO (II)
the product is isolated and is optionally converted to a salt with an inorganic or organic acid.

7. Use of the compounds of formula: 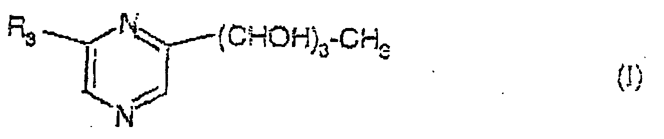 in which
R₃ represents a -CH₂-(CHOH)₂-CH₃ chain,
or one of their stereoisomers or one of their salts with an inorganic or organic acid in the preparation of pharmaceutical compositions of use in the treatment or prevention of diabetes and complications of diabetes.
